(19)

**Europäisches Patentamt**
**European Patent Office**
**Office européen des brevets**

(11) **EP 4 095 867 A1**

(12) **EUROPEAN PATENT APPLICATION**

(43) Date of publication:
**30.11.2022 Bulletin 2022/48**

(21) Application number: **21386030.7**

(22) Date of filing: **24.05.2021**

(51) International Patent Classification (IPC):
**G16H 50/20** (2018.01)   **G16B 30/00** (2019.01)
**G16B 40/20** (2019.01)   **G16H 50/70** (2018.01)

(52) Cooperative Patent Classification (CPC):
**G16B 40/20; G16B 30/00; G16H 50/20;
G16H 50/70**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Chatzaki, Ekaterini**
  **681 00 Alexandroupolis (GR)**
• **Democritus University of Trace**
  **681 00 Alexandroupolis (GR)**

(72) Inventors:
• **Chatzaki, Ekaterini**
  **681 00 Alexandroupolis (GR)**
• **Karaglani, Makrina**
  **681 00 Alexandroupolis (GR)**
• **Tsamardinos, Ioannis**
  **71305 Heraklion (GR)**

(74) Representative: **Vavekis, Konstantinos**
  **PO Box 1**
  **Mesarmos**
  **Voutes**
  **70013 Heraklion, Crete (GR)**

(54) **METHOD FOR MONITORING PANCREATIC BETA-CELL DESTRUCTION IN DISEASE PREDICTION/DIAGNOSIS/PROGNOSIS OF TYPE 2 DIABETES MELITUS**

(57)    The patent relates to a computer implemented method for early diagnosis of diabetes mellitus type 2 (T2DM) and β-pancreatic cell loss monitoring. The method employs Machine Learning tools to identify specific biomarkers related to circulating cell-free DNA (ccfDNA) to build accurate diagnostic/monitoring predictive biosignatures/models for clinical application. The models are further enriched with demographical/lifestyle/pathological/clinical subject data to achieve higher discriminating capacity. AutoML analysis of data delivered a five-feature biosignature, including GCK, IAPP and KCNJ11 gene methylation identified in ccfDNA and age and BMI, accurately discriminating T2DM patients from healthy individuals. This leads to a methylation-specific PCR based methodology for diagnosis/prognosis/monitoring of T2DM using liquid biopsy biomaterial.

FIG 1

**Description**

**Field of the invention**

The present invention relates to an innovative method for type 2 Diabetes Melitus (T2DM) prediciton/diagnosis/prognosis based on liquid biopsies and a specific computer implemented predictive model. In particular, the present invention relates to a novel method based on a machine learning-built model, intergrating diabetes-related/pancreatic beta-cell (β-cell) gene methylation detected in circulating cell-free DNA (ccfDNA) and selected lifestyle/clinical/demographical parameters, showing high sensitivity, specificity and accuracy in discriminating T2DM patients from healthy subjects. The method is capable of β-cell destruction detection and monitoring and can be used for early T2DM diagnosis, disease prediction and prognosis.

**Background of the invention**

T2DM is expected to be one of the leading causes of death globally by 2030, while it has been recognized globally as a serious pathological entity with 425 million adults having diabetes while the half remain undiagnosed(1). Therefore it is of crucial importance the early diagnosis of T2DM for provision of early and appropriate medical treatment to the suffering patients.

T2DM is characterized by inadequate β-cell function, insulin insensitivity and chronic inflammation, all of which progressively lead to impaired glucose homeostasis(2). In post-mortem specimens from patients with T2DM, β-cell mass is reduced by 30—40% compared with specimens from non-diabetic subjects(3). It is found that increased β-cell apoptosis and reduced functional capacity of the remaining cells are important factors that indicate progression of the disease(4). However, the detection of the loss of β-cell mass is currently possible after the development of hyperglycemia which means significant β-cell mass and therefore significant development of the disease limiting the possibility of early intervention.

Established biomarkers for monitoring T2DM progression like HbA1c levels are unable to detect ongoing β-cell destruction and its dynamics in islets. Thus, as the number of people with T2DM worldwide keeps on rising, there is an emerging need for additional more precise monitoring of β-cell death for disease prediciton, early diagnosis and leading therapeutic decisions.

The disclosed method is using the methylation profile of diabetes-related genes, assessed via methylation specific Polymerase Chain Reaction (PCR) or sequencing, to build a highly performing tool/model/classifier/biosignature of clinical value on diabetes. Integrating lifestyle/clinical/demographical data with ccfDNA gene methylation patterns in machine learning analysis allowed the formation of predictive models with high sensitivity and specificity in recognizing T2DM patients from healthy subjects.

**Prior art**

Several attempts have been made for early diagnosis of Diabetes and monitoring using minimally invasive biomarkers. Towards this direction, epigenetic information detected in ccfDNA is currently tested as a clinically valuable biomarker of pancreatic β-cell death in type 1 Diabetes Mellitus (T1DM) by several groups(5-12). These biomarkers rely on the principles that a. cells release amounts of their DNA into the bloodstream and/or other biofluids where they are easily accessible(13) and b. each cell type has a unique methylome that affects gene expression(14). Akirav's group (33), has identified a unique DNA CpG methylation pattern as compared to other cells in the blood upon pancreatic β-cell death. The method used comprises extracting and purifying DNA; treating the extracted purified DNA with bisulfite to convert cytosine to uracil; amplifying a region of *INS* gene on the bisulfite-treated DNA by PCR; determining a quantitative relationship between the DNA portion having the unique DNA CpG methylation pattern to the DNA portion lacking the unique DNA CpG methylation pattern, by employing DNA CpG methylation pattern-specific probes; and computing a difference between the DNA portion having the unique DNA CpG methylation pattern and the DNA portion lacking the unique DNA CpG methylation pattern. A general method of detecting death of a cell type or tissue of any type in a subject is disclosed from Dor (34), wherein a methylome atlas comprises methylation data from several cell types including pancreatic β-cells. The existing methods are based on the selection of a single methylation biomarker and this results to high uncertainty in a disease diagnosis including Diabetes.

Machine learning (ML) is the application of artificial intelligence on data analysis to build trained models (15). ML has penetrated biomarker discovery in many diseases(16-18) and in diabetes(19, 20). ML had been used for building models useful for the prediction and diagnosis of diseases such as Alzheimer's disease(21), lung and breast cancer(22, 23). ML has been used previously (35) to form a risk predictive model of T2DM based on data from insurance claim databases. However, the model does not disclose the specific data used or a specific model but it is rather used as a general framework analyzing any of the given data. The method needs many data and the accuracy of disease detection is low as compared to the biomarker-based methods.

**Summary of the invention**

The present invention was made in view of the prior art described above and the object of the present invention is to provide a method of improved performance over the prior art in detecting efficiently pancreatic β-cell damage and as such capable of early diagnosis, prognosis, prediction or monitoring of T2DM, reliably and with higher sensitivity/specificity/accuracy that existing methods.

To solve the problem, we focus on liquid biopsy experimental parameters related to gene methylation detected in ccfDNA isolated from the blood of T2DM patients and healthy subjects. The levels of ccfDNA were directly quantified in serum by fluoromentry. Methylation of five diabetes-related β-pancreatic cell genes used as biomarkers, namely *INS* (insulin), *IAPP* (Islet Amyloid Polypeptide-Amylin), *GCK* (Glucokinase), *KCNJ11* (Potassium Inwardly Rectifying Channel Subfamily J Member 11) and *ABCC8* (ATP Binding Cassette Subfamily C Member 8) were detected via quantitative SYBR Green-based methylation specific PCR. Then, ad hoc **Automated ML (AutoML)** technology (JADBio) was applied on the ccfDNA experimental parameters to build accurate diagnostic/monitoring predictive models/biosignatures/classifiers of clinical value on diabetes. An exceptionally high classifying performance/predicting capacity as compared to the prior art, was achieved when specific 2 or 3 of the above biomarkers were combined. In specific, it was found that the inclusion of *GCK* methylation and one or both from *IAPP* and *KCNJ11* methylation provided significantly higher discrimination performace between T2DM patients and healthy subjects [three feature model's AUC 0.892 (95%CI 0.848 - 0.934)] as compared to *GCK* methylation alone [AUC 0.849 (95%CI 0.803 - 0.892)] or combined with other biomarkers. In a further ML analysis, we combined these ccfDNA experimental biomarkers with lifestyle/clinical/demographical data (i.e., age, gender, Body Mass Index -BMI, smoking etc.) and the predictive capacity of the resulting models was further improved. The best predictive model found was based on a best performing five-feature biosignature selected via Classification Random Forests algorithm, including *GCK, IAPP* and *KCNJ11* methylation and age and BMI, showing high performance in discriminating between T2DM patients and healthy subjects with an AUC of 0.927 (95%CI 0.874 - 0.967) and an average Precision of 0.951 (95% CI 0.914 - 0.980). A best interpretable model of five features was also built via Ridge Logistic Regression algorithm reaching an AUC of 0.915 (95% CI 0.868 - 0.957) and an average Precision of 0.941 (95% CI 0.901 - 0.975). This biosignature 's features included *GCK, IAPP* and *KCNJ11* methylation, smoking status and BMI.

Comparison of the results to existing studies showed that models built here, including *GCK* methylation combined with other parameters from the above, are superior in T2DM prediction performance than any other reported so far in the prior art.

**Brief Description of Drawings**

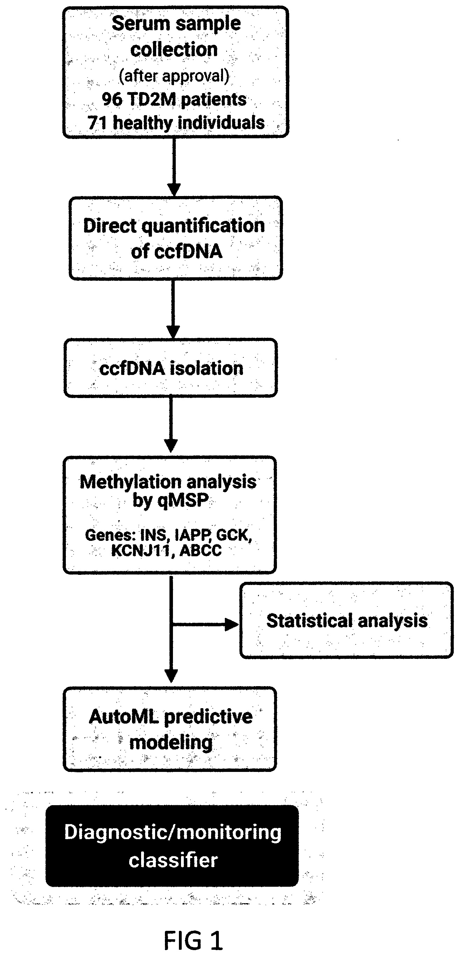

Figure 1 Workflow of the disclosed study.

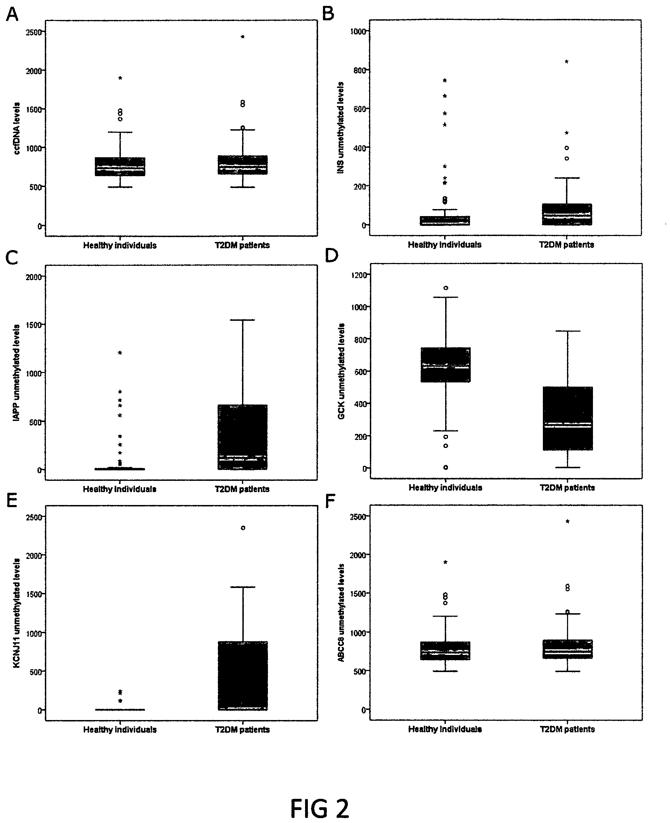

Figure 2 A. Box plot of serum ccfDNA levels between healthy subjects and T2DM patients *(p=0.552)*. B. Box plot of *INS* methylation between healthy subjects and T2DM patients (*p=0.001*). C. Box plot of *IAPP* methylation between healthy subjects and T2DM patients (*p<0.001*). D. Box plot of *GCK* methylation between healthy subjects and T2DM patients (p<0.001). E. Box plot of *KCNJ11* methylation between healthy subjects and T2DM patients (*p=0.001*). F. Box plot of *ABCC8* methylation between healthy subjects and T2DM patients (*p=0.534*).

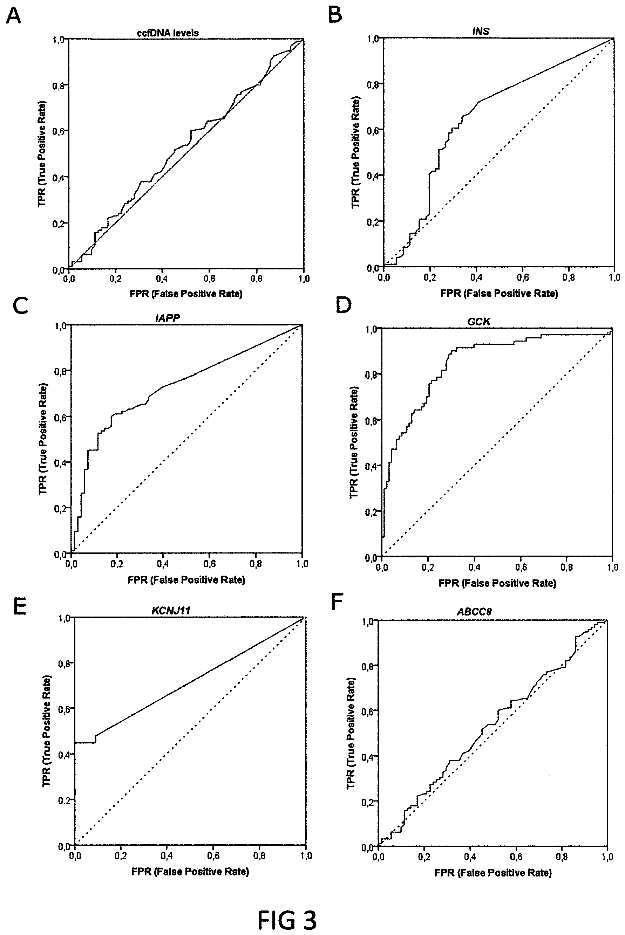

Figure 3 A. ROC curve of ccfDNA levels reaching an AUC of 0.527 (95%CI 0.438 - 0.616). B. ROC curve *of INS* gene reaching an AUC of 0.650 (95%CI 0.562 - 0.737). C. ROC curve of *IAPP* gene reaching an AUC of 0.727 (95%CI 0.649 - 0.805). D. ROC curve of *GCK* gene reaching an AUC of 0.848 (95%CI 0.787 - 0.910). E. ROC curve of *KCNJ11* gene reaching an AUC of 0.712 (95%CI 0.619 - 0.806). F. ROC curve of *ABCC8* gene reaching an AUC of 0.528 (95%CI 0.439 - 0.617).

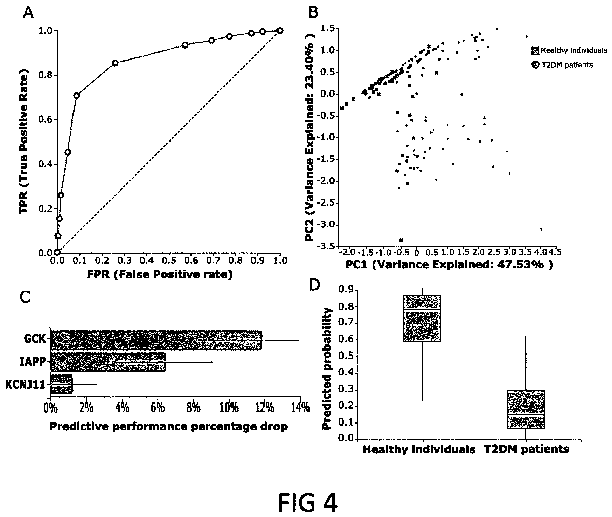

Figure 4 Predictive modelling results of the three-feature *(GCK, IAPP* and *KCNJ11* methylation) best interpretable model A. ROC curve reaching an AUC of 0.892 (95%CI 0.848 - 0.934), B. Principal Component Analysis (PCA) plot depicting discrimination between T2DM patients and healthy subjects, C. Feature Importance plot of the features of the model. Feature importance is defined as the percentage drop in predictive performance when the feature is removed from the model, D. Probabilities box plot of out-of-sample predictions.

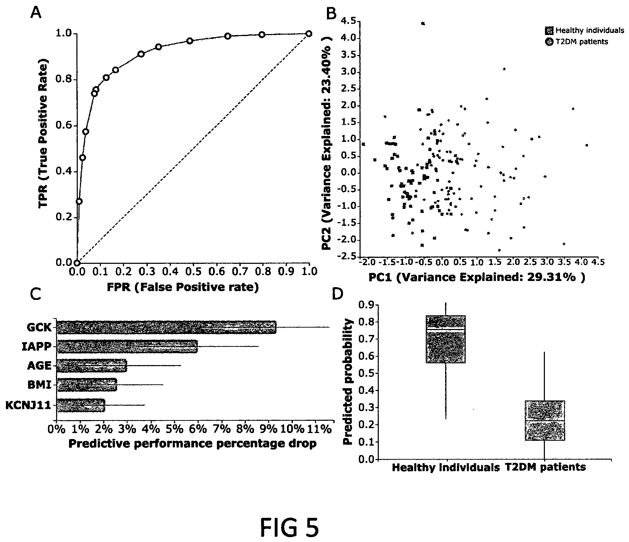

Figure 5 Predictive modelling results of the five-feature *(GCK, IAPP* and *KCNJ11* methylation and age and BMI) best perfoming model A. ROC curve reaching an AUC of 0.927 (95%CI 0.874 - 0.967), B. PCA plot depicting discrimination between T2DM patients and healthy subjects, C. Feature Importance plot of the features of the model. Feature importance is defined as the percentage drop in predictive performance when the feature is removed from the model, D. Probabilities box plot of out-of-sample predictions.

## Detailed description

The development of the method was based on a cohort of T2DM patients which was compared to a group of healthy subjects.

In one embodiment a computer implemented method suitable for monitoring pancreatic β-cell destruction of value in disease prediction, prognosis and early diagnosis of T2DM, was developed, said method comprising:

- training a machine learning tool to minimize false positives and to maximize true positives using a training dataset,
- said training dataset including data from patients diagnosed with T2DM and healthy subjects,
- using said machine learning tool to create a prediction model that predicts, given at least two or more input parameters, onset or progression of T2DM,
- wherein the input parameters comprise at least of two methylation gene related biomarkers, and
- storing the trained machine learning tool in at least one computer memory or on the cloud,

In a further embodiment the above Computer Implemented method for monitoring pancreatic β-cell destruction is used and said biomarkers are obtained by a method comprising:

- Providing a biological fluid sample from a subject suspected of having or developing or diagnosed with said disease or a subject known not to have the said disease
- Directly quantifying circulating cell free DNA (ccfDNA)
- Extracting ccfDNA
- Treating the said DNA to convert demethylated cytosines to uracils while sparing the methylated cytosines
- Performing a methylation specific detection to measure diabetes-related gene methylation
- Incorporate the said methylation measurements of diabetes-related genes to the trained machine learning tool as the input parameters.

In further embodiments, different Machine learning tools (machine learning classification algorithms) are used to build the predictive models. Any machine learning classification classification algorithm can be used and in different embodiments the following: Decision Tree, k-Nearest Neighbors (k-NN), Gradient Boosting Machine (GBM), linear kernel Support Vector Machine (SVM-linear), Radial Basis Function (RBF) kernel Support Vector Machine, Artificial Neural Network (ANN), Multifactor Dimensionality Reduction (MDR), naive Bayes, Classification And Regression Tree (CART) and preferably Support Vector Machine (SVM) or (Classification) Random Forest (RF) or Logistic Regression (LR).

In one embodiment the biological sample used to obtain some of the biomarkers in the above methods comprises a body fluid and in a further embodiment blood sample and in further embodiments serum or plasma and combinations thereof.

In further embodiments, in the above computer implemented methods the biomarkers used to train the Machine learning tools are diabetes-related genes, in further embodiments are pancreatic β-cell genes and in further embodiments the genes *GSK* and/or *IAPP* and/or *KCNJ11* and combinations thereof.

In a further embodiment the above prediction model has an AUC (Area under Curve) of 0.884 or higher indicating the high predictive capability of the model.

In further embodiments the said methylation measurements of the genes are expressed either qualitatively or quantitatively as indexes of methylation levels and in further embodiments by any of the following quantification methods/formulas and combinations thereof

- The relative quantity (RQsample) of demethylated alleles using the $2^{-\Delta\Delta CT}$ method where the $\Delta\Delta CT$ values were generated for each target after normalization by reference gene values.

- The demethylation index was calculated using the formula: $2^{(\text{methylated Ct} - \text{unmethylated Ct})}$.

- The percentage of methylation in a sample using the formula: $1 - \dfrac{1}{1+2^{(-\Delta Ct)}} \times 100\%$ , where $\Delta Ct = Ct_{\text{unmethylated}} - Ct_{\text{methylated}}$

In further embodiments the reference gene is any housekeeping gene and in further embodiments the reference genes used are *ACTB* or *GADPH* or *COL2A1* gene respectively.

In one embodiment the methylation specific detection to measure diabetes-related gene methylation and its levels is conducted by sequencing and in a further embodiment by PCR-based technology.

In further embodiments the input parameters further comprising lifestyle and/or personal and/or demographic and/or clinical and/or clinicopathological data of the subjects and in additional embodiments, the BMI and/or the smoking status and /or the age of the subjects and combinations thereof.

In a further embodiment a method is used to test the biomarkers of a subject on the previous trained predictive model to early diagnose / predict the development of type 2 diabetes mellitus on the said subject. The method comprises of the steps

- Providing a biological fluid sample from a subject suspected of having or having said disease
- Directly quantifying circulating cell free DNA (ccfDNA)
- Extracting ccfDNA
- Treating the said DNA to convert demethylated cytosines to uracils while sparing the methylated cytosines
- Performing a methylation specific detection based on PCR or sequencing-based technology and in further embodiments quantitative methylation specific PCR, to measure the methylation of diabetes-related genes and in further embodiments pancreatic β-cell genes and in further embodiments of the genes *GSK* and/or *IAPP* and/or *KCNJ11* and combinations thereof
- Incorporate input data comprising of measurements of the amount of methylation of at least two of the said selected genes as data input parameters into the trained predictive model built by the above predictive machine learning tool
- Produce a score using the trained machine learning tool of the previous embodiments, indicative for clinical prognosis or diagnosis.

In this embodiments the data used are in accordance to the data input required in the predictive model.

In a further embodiment the biological sample used to obtain some of the biomarkers of the subject to be tested on the trained predictive model comprises a body fluid and in a further embodiment blood sample and in further embodiments serum or plasma and combinations thereof

In a further embodiment the input data of the biomarkers of the subject to be tested on the trained predictive model comprises further of the lifestyle and/or personal and/or demographic data and/or clinical and/or clinicopathological data of the subject and in further embodiments the BMI and/or the smoking status and /or the age of the subjects and combinations thereof and in accordance to the predictive model.

In further embodiments the input data of the biomarkers of the subject to be tested on the trained predictive model comprises of the said methylation measurements of the genes, expressed either qualitatively or quantitatively as indexes of methylation levels and in further embodiments by any of the following quantification methods/formulas and combinations thereof and in accordance to the methods/formulas used for the training of the predictive model.

- The relative quantity (RQsample) of demethylated alleles using the 2-ΔΔCT method where the ΔΔCT values were generated for each target after normalization by reference gene values.
- The demethylation index was calculated using the formula: 2(methylated Ct - unmethylated Ct).
- The percentage of methylation in a sample using the formula: $1- 1/(1+2^{((-\Delta Ct)})) \times 100\%$, where ΔCt=Ctunmethylated-Ctmethylated

In a further embodiment a computer implemented method was used comprising of the input of the biomarkers of the tested for diabetes T2DM having or going to develop subject in the trained predictive model as manual input dataset or as data stored in a database or a non-transient computer memory in order the predictive model to produce a score using the trained machine learning tool of the previous embodiments, indicative for clinical prognosis or diagnosis.

In a specific embodiment, the study's groups consisted of 96 T2DM patients on treatment and 71 healthy subjects of similar age without history of diabetes. All samples were of Caucasian origin. T2DM was diagnosed according to the American Diabetes Association (ADA) guidelines(24). Lifestyle/personal/clinical/demographic data of study groups are shown in Table 1.

*Table 1 Lifestyle/personal/clinical/demographic data of study groups.*

| | T2DM patients (n=96) | Healthy subjects (n=71) |
|---|---|---|
| **Gender (male)** | 58 (60) | 50 (70) |
| **Age (years)** | 64 ±8 | 62 ±10 |

(continued)

| | T2DM patients (n=96) | Healthy subjects (n=71) |
|---|---|---|
| BMI (kg/m$^2$) | 30.9 ±4.7 | 28.4 ±5.2 |
| Smoking | 16 (17) | 25 (39) |
| Glucose (mg/dL) | 138 ±37 | 85 ±13 |
| C-peptide (ng/mL) | 4.1 ±3.2 | - |
| HbA1c (%) | 7.2 ±1.1 | - |
| Diabetes duration (years) | 14 ±9 | - |
| Diabetes complication (presence) | 25 (26) | - |
| Diabetes therapy (yes) | 96 (100) | - |
| Oral therapy (metformin, etc.) | 96 (100) | - |
| Insulin | 42 (44) | - |

Inclusion criteria of the study included age more than 25 and up to 75 years old and ability to give informed consent. Exclusion criteria of all participants included the presence of a (another) chronic disease, underlying malignancies and systemic lupus erythematosus.

ccfDNA was isolated and measured in body fluids. In a specific embodiment, serum samples were obtained within 2 hours of blood sampling through centrifugation at 3,000×g for 10min. An additional high-speed centrifugation step at 14,000×g for 10min was performed to remove any cellular debris and contaminants, like gDNA from damaged blood cells. Serum samples were stored at -80°C until further use. Following, ccfDNA was quantified. Several direct or indirect methods can be used for quantifying ccfDNA in body fluids with comparable results. In a specific embodiment, ccfDNA was directly quantified in 20μL of serum, utilizing the Quant-iT™ dsDNA High-Sensitivity Assay Kit in Qubit® 3.0 Fluorometer (Invitrogen, Karlsruhe, Germany). A standard curve was generated using kit provided standards (0ng/μl and 10ng/μl). Experimental data from measured ccfDNA levels are shown in Figure 2A. ccfDNA levels did not differ between healthy subjects and diabetes patients [700(400 - 1590) ng/ml vs. 820(490 - 2430) ng/ml, respectively, $p$=0.552]

Following, ccfDNA was extracted from body fluids. Several methods can be used. In a specific embodiment, ccfDNA was extracted from 600μl of serum using the QIAamp® Blood Mini kit (Qiagen, Hilden, Germany) in a final elution volume of 30μl. The extracted ccfDNA was stored at -20°C until further use.

*Table 2 Primer sequences, genomic locations, and related references (where relevant) used for PCR assays.*

| Primer name | Primer sequence (5'-3') | Genomic Location (GRCh38) | Ref. |
|---|---|---|---|
| ACTBF | TGG TGA TGG AGG AGG TTT AGT AAG T | 7:5,558,705:-1 | (25) |
| ACTBR | AAC CAA TAA AAC CTA CTC CTC CC | 7:5,558,838:-1 | |
| INSMETHF | **CGG** AAA TTG TAG TTT TAG TTT TTA GTT ATT TGT C | 11:2,159,776:-1 | |
| INSMETHR | CCT AAA AAA CTA AAA ACT ACT AAA CCC CCG | 11:2,159,922:-1 | |
| INSUNMETHF | TGG AAA TTG TAG TTT TAG TTT TTA GTT ATT TGT T | 11:2,159,776:-1 | |
| INSUNMETHR | CCT AAA AAA CTA AAA ACT ACT AAA CCC CCA | 11:2,159,922:-1 | |
| IAPPF | TGT TAT TAG TTA TTA GGT GGA AAA G | 12:21,378,228:1 | |
| IAPPMETHR | TAA AAA ATT TAC CAA ACG CTA CG | 12:21,378,304:1 | (8) |
| IAPPUNMETHR | TAA AAA ATT TAC CAA ACA CTA CA | 12:21,378,304:1 | |
| GCKMETHF | AAT GTC GAG CGG CGT TTG AG | 7:44,153,026:-1 | |
| GCKUNMETHF | AGG AAA TGT TGA GTG GTG TTT GAG T | 7:44,153,022:-1 | (9) |
| GCKR | ATC CTC TCC CTT CTA TAA CCT AAA AAC AAC | 7:44,153,136:-1 | |
| KCNJ11METHF | CGG GTT TCG GTT TCG TTC | 11:17,389,140:1 | |
| KCNJ11METHR | CAC GAA CGA ACA AAC AAA CG | 11:17,389,253:1 | |
| KCNJ11UNMETHF | TGG GTT TTG GTT TTG TTT GTT GT | 11:17,389,140:1 | |
| KCNJ11UNMETHR | ACC CAC AAA CAA ACA AAC AAA CA | 11:17,389,254:1 | |

(continued)

| Primer name | Primer sequence (5'-3') | Genomic Location (GRCh38) | Ref. |
|---|---|---|---|
| ABCC8METHF | TAG GAA GAC GTG CGG TAT TAC | 11:17,476,660:1 | |
| ABCC8METHR | CTA CGA CAA CGA AAA CCA CT | 11:17,476,743:1 | |
| ABCC8UNMETHF | GAG TAG GAA GAT GTG TGG TAT TAT | 11:17,476,657:1 | |
| ABCC8UNMETHR | CTT CTA CAA CAA CAA AAA CCA CT | 11:17,476,743:1 | |

*F forward, R reverse, METH methylated, UNMETH unmethylated*

Following, gene methylation was detected in ccfDNA after converting by sodium bisulfite. In one embodiment, 20μl of extracted ccfDNA were treated with sodium bisulfite (SB) using the EZ DNA Methylation-Gold™ kit (ZYMO Research Co., CA, USA) in a final elution volume of 10μl. In each reaction, CpGenome™ Human methylated and non-methylated DNA controls (Merck Millipore, Darmstadt, Germany) were included as negative and positive control samples, respectively. The SB-treated ccfDNA was stored at -80°C until further use.

Different PCR-based (i.e. methylation-specific PCR MSP, Methylight and others) or sequencing-based (i.e. bisulfite sequencing, pyrosequencing and others) methodologies are available for detecting gene methylation on DNA. In one embodiment, a methylation-independent PCR assay for the housekeeping gene β-actin (*ACTB*) was used in order to verify quality and quantity of SB-treated ccfDNA. Several other housekeeping genes can be used i.e. the Glyceraldehyde-3-Phosphate Dehydrogenase *(GAPDH)* gene or the Collagen Type II Alpha 1 Chain (*COL2A1*) gene. Following, methylation status (qualitative measurements) and methylation levels (quantitative measurements) of diabetes-related genes, i.e. *INS, IAPP, GCK, KCNJ11* and *ABCC8* were analyzed using methylation-dependent SYBR Green-based PCR (MSP) assays. For all genes, primers specific for methylated (m) and unmethylated (u) alleles were either newly designed using the MethPrimer(26) program or were based on bibliography with some modifications. Primer sequences are provided in Table 2. Assay conditions are presented in Table 3.

*Table 3 Assay conditions*

| Assay | Annealing temperature (°C) meth/unmeth |
|---|---|
| ACTB | 60 |
| INS | 62/ 62 |
| IAPP | 62/ 60 |
| GCK | 65/ 65 |
| KCNJ11 | 58/ 60 |
| ABCC8 | 58/ 58 |

*METH methylated, UNMETH unmethylated*

Analytical sensitivity of developed PCR assays was evaluated using serial dilutions of SB-treated methylated and non-methylated DNA controls while efficiency using serial dilutions of the SB-treated DNA controls in H$_2$O. The analytical sensitivity of all our developed assays was found to be 0.1 % in the detection of methylated DNA molecules in a background of unmethylated DNA and 0.5% in the detection of unmethylated DNA molecules in a background of methylated DNA. The efficiency of all our developed assays ranged between 93-96%.

Quantification of methylation can be implemented by different formulas/methods. In one embodiment, methylation in a sample was estimated using the formula: $1-\dfrac{1}{1+2^{(-\Delta Ct)}} \times 100\%$, where $\Delta Ct=Ct_{Unmethylated}-Ct_{methylated}$(27). Following, the percentage of methylation was multiplied by the concentration of total serum ccfDNA.

In other embodiments we have used alternative formulas for the quantification of methylation of the selected genes with similar results:

- The relative quantity (RQ$_{sample}$) of demethylated alleles using the 2$^{-\Delta\Delta CT}$ method where the ΔΔCT values were generated for each target after normalization by reference gene values(28).

- The demethylation index was calculated using the formula: 2$^{(methylated Ct - demethylated Ct)}$ (5). The acquired data referring to experimental liquid biopsy parameters (including diabetes-related gene methylation and ccfDNA parameters) as well as the lifestyle/clinical/personal/demographic subject data, forming a 2D matrix (i.e. samples/subjects in rows, parameters in columns), were stored in computer memory storage or in cloud to be analyzed. Alternatively, the data can be inserted manually in a computer software.

The results obtained were initially tested using the Kolmogorov-Smirnov test to check for normality in distribution of continuous data. Due to lack of normality in data, non-parametric statistics were used for comparisons between groups. The Spearman correlation coefficient was used as a measurement of correlation for continuous variables. The predictive power of the experimental liquid biopsy parameters was tested using Receiver operating characteristic (ROC) curve analysis and Area Under the Curve (AUC) metric. In all tests performed, statistical significance was set at two-sided p value <0.050. Standard statistical analysis was carried out with the SPSS version 21.0 statistical software package for Windows (IBM - SPSS Inc., USA).

*INS, IAPP, GCK* and *KCNJ11* methylation was significantly different in T2DM patients as compared to the healthy subjects (*p*=0.001, *p*<0.001, *p*<0.001 and *p*<0.001, respectively) while *ABCC8* methylation didn't differ between groups (p=0.534) (Figure 2). ROC curve analysis showed that *GCK* methylation could provide very good discrimination between T2DM patients and healthy subjects (AUC 0.848 [95%CI 0.787 - 0.910]) (Figure 3D) while *IAPP* and *KCNJ11* methylation could offer good discrimination between groups (AUC 0.727 [95%CI 0.649 - 0.805] and AUC 0.712 [95%CI 0.619 - 0.806], respectively) (Figure 3C and E). *INS* methylation showed poor discrimination capacity between patients and controls (AUC 0.650 [95%CI 0.562 - 0.737]) (Figure 3B).

Following, data were analyzed by ML tools. Multiple ML algorithms have been employed to build models/classifiers/biosignatures over our dataset to discriminate T2DM from healthy subjects. In one embodiment, our novel liquid biopsy-based data were analyzed by an innovative AutoML tool, namely JADBio version 1.2.8 (https://www.jadbio.com/)(29) in order to produce diagnostic/monitoring models of clinical value. The JADBio tool employed for this analysis performs and compares automatically all standard, best-practices, and advanced ML techniques and produces the optimal best performing model along with the best interpretable one(29). Given a 2D matrix of data, JADBio automatically preprocesses data (Mean Imputation, Mode Imputation, Constant Removal, Standardization), performs feature selection by employing LASSO or Statistical Equivalent Signatures (SES) algorithms, tries several algorithms (i.e. Classification Random Forests, Support Vector Machines (SVM), Ridge Logistic Regression and Classification Decision Trees) and thousands of algorithmic configurations, selects the best performing model and estimates the out-of-sample model's performance after bootstrap correction and cross validation and provides several visualizations(29). For our AutoML classification analysis, we used extensive model tuning effort, we chose the AUC metric for optimization of performance and we set classifier maximum size to five features. The predictive power of the model was assessed using AUC and average Precision (aka area under the Precision-Recall curve) metrics.

In one embodiment, AutoML technology JADBio, was applied to produce diagnostic/monitoring tools/models/biosignatures/classifiers based on the ccfDNA experimental parameters, diabetes-related gene methylation and lifestyle/personal/clinical/demographic subject data.

The AutoML technology JADBio also conducted statistical analysis based on logistic regression model and the results confirmed the models built by the other ML algorithms.

We used AutoML analysis, to predict T2DM versus health from the acquired liquid biopsy parameters (diabetes-related gene methylation and ccfDNA parameters) without and with the lifestyle/personal/clinical/demographic subject data (age, gender, BMI, etc.). In the former case, models build including *GCK* gene methylation detected in ccfDNA plus at least one other pancreatic gene methylation, demonstrated significantly improved discriminating performance. In specific, the best performing combinations were i) *GCK* and *IAPP,* ii) *GCK* and *KCNJ11*, iii) *GCK, KCNJ11* and *IAPP* (Figure 4), showing high performance with AUC ranging from 0.849 (95%CI 0.803 - 0.892) to 0.892 (95%CI 0.848 - 0.934).

In the latter case, when liquid biopsy parameters (diabetes-related gene methylation and ccfDNA parameters) were combined with subjects' lifestyle/personal/clinical/demographic data, discriminating capacity of AutoML built models was even higher. This AutoML analysis produced a best performing five-feature biosignature via Classification Random Forests algorithm that was able to discriminate extremely well between diabetes patients and healthy subjects with an AUC of 0.927 (95%CI 0.874 - 0.967) and an average Precision of 0.951 (95% CI 0.914 - 0.980). Biosignature's features included *GCK, IAPP* and *KCNJ11* methylation as well as age and BMI. Best performing biosignature performance is presented in Figure 5. A best interpretable five-feature biosignature was also built via Ridge Logistic Regression algorithm reaching an AUC of 0.915 (95% CI 0.868 - 0.957) and an average Precision of 0.941 (95% CI 0.901 - 0.975). This biosignature's features included *GCK, IAPP* and *KCNJ11* methylation, smoking status and BMI.

This discriminating performance is higher than any other previously reported or used generally in diabetes pathological entities. For example, *IAPP* methylation was able to discriminate recent onset T1DM patients and age-matched unrelated healthy subjects with an AUC of 0.866 (95%CI 0.720 - 1.010)(8). Other studies leveraged demographical, clinical and laboratory data such as age, sex, BMI, glycated hemoglobin (HbA1c), hypertension, smoking, glucose etc. and employed ML tools for predicting diabetes with promising results(19, 20, 30, 31). The predictive ability of the models built however (AUC ranging from 0.826 to 0.872) were inferior to those presented here (AUC reaching 0.927). Only the recent publication of Kaur et al. that analyzed the PIMA Indian Diabetes dataset consisted only of female patients, produced five different predictive models with AUC range from 0.850 - 0.920(32). The superiority of our model here relies on the use of both sexes in our cohort, and the incorporation of experimental beta-cell specific gene parameters, suitable to detect and monitor beta-cell death.

In addition, it had been shown that *GCK* methylation can present an early biomarker for β-pancreatic cell destruction(9). Our model incorporates this biomarker with the maximal feature importance in the model's performance, which however is dramatically increased by including also other selected pancreatic gene methylation biomarkers and other parameters. These corroborated findings considered together, support the value of the herein model in the early detection of diabetes onset.

In further embodiments, biomarkers from a subject suspected of having or having said disease are used as input parameters to the trained as previously mentioned prediction models to produce a score indicative for clinical prognosis or diagnosis of Diabetes T2DM. The biomarkers used as input were the same in number and type used to train the predictive model.

The input data in one embodiment are resulting from a method comprising of:

- Providing a biological fluid sample from a subject suspected of having or having said disease
- Directly quantifying circulating cell free DNA (ccfDNA)
- Extracting ccfDNA
- Treating the said DNA to convert demethylated cytosines to uracils while sparing the methylated cytosines
- Performing a methylation specific detection based on PCR or sequencing-based technology and preferably quantitative methylation specific PCR, to measure the methylation of diabetes-related genes and preferably pancreatic β-cell genes and more preferably of the genes *GSK* and/or *IAPP* and/or *KCNJ11* and combinations thereof

In further embodiments the data are stored in at least one non-transitory processor-readable storage medium on a computer system or on the cloud and used as input in the predictive model.

References

1. Saeedi P, Petersohn I, Salpea P, Malanda B, Karuranga S, Unwin N, et al. Global and regional diabetes prevalence estimates for 2019 and projections for 2030 and 2045: Results from the International Diabetes Federation Diabetes Atlas, 9th edition. Diabetes research and clinical practice. 2019; 157.

2. Kharroubi AT, Darwish HM. Diabetes mellitus: The epidemic of the century. World J Diabetes. 2015;6(6):850-67.

3. Rahier J, Guiot Y, Goebbels RM, Sempoux C, Henquin JC. Pancreatic beta-cell mass in European subjects with type 2 diabetes. Diabetes, obesity & metabolism. 2008;10 Suppl 4:32-42.

4. Butler AE, Janson J, Bonner-Weir S, Ritzel R, Rizza RA, Butler PC. Beta-cell deficit and increased beta-cell apoptosis in humans with type 2 diabetes. Diabetes. 2003;52(1):102-10.

5. Akirav EM, Lebastchi J, Galvan EM, Henegariu O, Akirav M, Ablamunits V, et al. Detection of beta cell death in diabetes using differentially methylated circulating DNA. Proceedings of the National Academy of Sciences of the United States of America. 2011;108(47):19018-23.

6. Husseiny MI, Kaye A, Zebadua E, Kandeel F, Ferreri K. Tissue-specific methylation of human insulin gene and PCR assay for monitoring beta cell death. PloS one. 2014;9(4):e94591.

7. Herold KC, Usmani-Brown S, Ghazi T, Lebastchi J, Beam CA, Bellin MD, et al. beta cell death and dysfunction during type 1 diabetes development in at-risk individuals. The Journal of clinical investigation. 2015;125(3):1163-73.

8. Olsen JA, Kenna LA, Spelios MG, Hessner MJ, Akirav EM. Circulating Differentially Methylated Amylin DNA as a Biomarker of beta-Cell Loss in Type 1 Diabetes. PloS one. 2016;11(4):e0152662.

9. Sklenarova J, Petruzelkova L, Kolouskova S, Lebl J, Sumnik Z, Cinek O. Glucokinase Gene May Be a More Suitable Target Than the Insulin Gene for Detection of beta Cell Death. Endocrinology. 2017;158(7):2058-65.

10. Lehmann-Werman R, Neiman D, Zemmour H, Moss J, Magenheim J, Vaknin-Dembinsky A, et al. Identification of tissue-specific cell death using methylation patterns of circulating DNA. Proceedings of the National Academy of Sciences of the United States of America. 2016;113(13):E1826-34.

11. Usmani-Brown S, Lebastchi J, Steck AK, Beam C, Herold KC, Ledizet M. Analysis of beta-cell death in type 1 diabetes by droplet digital PCR. Endocrinology. 2014;155(9):3694-8.

12. Fisher MM, Perez Chumbiauca CN, Mather KJ, Mirmira RG, Tersey SA. Detection of islet beta-cell death in vivo by multiplex PCR analysis of differentially methylated DNA. Endocrinology. 2013;154(9):3476-81.

13. Gahan PB. Biology of circulating nucleic acids and possible roles in diagnosis and treatment in diabetes and cancer. Infectious disorders drug targets. 2012;12(5):360-70.

14. Bergman Y, Cedar H. DNA methylation dynamics in health and disease. Nature structural & molecular biology. 2013;20(3):274-81.

15. Davenport T, Kalakota R. The potential for artificial intelligence in healthcare. Future healthcare journal. 2019;6(2):94-8.

16. Myszczynska MA, Ojamies PN, Lacoste AMB, Neil D, Saffari A, Mead R, et al. Applications of machine learning to diagnosis and treatment of neurodegenerative diseases. Nature Reviews Neurology. 2020;16(8):440-56.

17. Schaefer J, Lehne M, Schepers J, Prasser F, Thun S. The use of machine learning in rare diseases: a scoping review. Orphanet Journal of Rare Diseases. 2020;15(1):145.

18. Kourou K, Exarchos TP, Exarchos KP, Karamouzis MV, Fotiadis DI. Machine learning applications in cancer prognosis and prediction. Computational and Structural Biotechnology Journal. 2015;13:8-17.

19. Zhang L, Wang Y, Niu M, Wang C, Wang Z. Machine learning for characterizing risk of type 2 diabetes mellitus in a rural Chinese population: the Henan Rural Cohort Study. Scientific Reports. 2020;10(1):4406.

20. Muhammad LJ, Algehyne EA, Usman SS. Predictive Supervised Machine Learning Models for Diabetes Mellitus. SN Computer Science. 2020;1(5):240.

21. Karaglani M, Gourlia K, Tsamardinos I, Chatzaki E. Accurate Blood-Based Diagnostic Biosignatures for Alzheimer's Disease via Automated Machine Learning. Journal of clinical medicine [Internet]. 2020 2020/09//; 9(9).

22. Markaki M, Tsamardinos I, Langhammer A, Lagani V, Hveem K, Røe OD. A Validated Clinical Risk Prediction Model for Lung Cancer in Smokers of All Ages and Exposure Types: A HUNT Study. EBioMedicine. 2018;31:36-46.

23. Panagopoulou M, Karaglani M, Balgkouranidou I, Biziota E, Koukaki T, Karamitrousis E, et al. Circulating cell-free DNA in breast cancer: size profiling, levels, and methylation patterns lead to prognostic and predictive classifiers. Oncogene. 2019;38(18):3387-401.

24. American Diabetes Association. 2. Classification and Diagnosis of Diabetes. Diabetes Care. 2015;38(Supplement 1):S8.

25. Hattermann K, Mehdorn HM, Mentlein R, Schultka S, Held-Feindt J. A methylation-specific and SYBR-green-based quantitative polymerase chain reaction technique for O6-methylguanine DNA methyltransferase promoter methylation analysis. Analytical biochemistry. 2008;377(1):62-71.

26. Li LC, Dahiya R. MethPrimer: designing primers for methylation PCRs. Bioinformatics (Oxford, England). 2002;18(11):1427-31.

27. Lu L, Katsaros D, de la Longrais IA, Sochirca O, Yu H. Hypermethylation of let-7a-3 in epithelial ovarian cancer is associated with low insulin-like growth factor-II expression and favorable prognosis. Cancer research. 2007;67(21):10117-22.

28. Livak KJ, Schmittgen TD. Analysis of relative gene expression data using real-time quantitative PCR and the 2(-Delta Delta C(T)) Method. Methods (San Diego, Calif). 2001;25(4):402-8.

29. Tsamardinos I, Charonyktakis P, Lakiotaki K, Borboudakis G, Zenklusen JC, Juhl H, et al. Just Add Data: Automated Predictive Modeling and BioSignature Discovery. bioRxiv. 2020:2020.05.04.075747.

30. Farran B, Channanath AM, Behbehani K, Thanaraj TA. Predictive models to assess risk of type 2 diabetes, hypertension and comorbidity: machine-learning algorithms and validation using national health data from Kuwait—a cohort study. 2013;3(5):e002457.

31. Lai H, Huang H, Keshavjee K, Guergachi A, Gao X. Predictive models for diabetes mellitus using machine learning techniques. BMC Endocrine Disorders. 2019;19(1):101.

32. Kaur H, Kumari V. Predictive modelling and analytics for diabetes using a machine learning approach. Applied Computing and Informatics. 2020.

33. US20160369340A1, Winthrop University Hospital, 2016-12-22.

34. US20210087630A1, Yissum Research Development Co Of Hebrew University Of Jerusalmem Ltd Hadasit Medical Research Services and Development Co Yissum Research Development Company of Hebrew University of Jerusalem, 2021-3-25

35. US20170308981A1, Blecker Saul; INDEPENDENCE BLUE CROSS; NEW YORK UNIVERSITY; Nigam Somesh; Razavian Narges Sharif; Schmidt Ann Marie; Smith-McLallen Aaron; Sontag David, 2017-10-26

## Claims

1. A computer implemented method for monitoring pancreatic β-cell destruction indicative of prediction, prognosis and diagnosis of type 2 Diabetes Mellitus (T2DM), said method comprising:

   - training a machine learning tool to minimize false positives and to maximize true positives using a training dataset,
   - said training dataset including data from patients diagnosed with T2DM and healthy subjects,

- using said machine learning tool to create a prediction model that predicts, given at least two or more input parameters, onset or progression of T2DM,
- and storing the trained machine learning tool in at least one non-transitory processor-readable storage medium on a computer system or on the cloud,

wherein the input parameters comprise at least of two methylation gene related biomarkers obtained by the method comprising:

- Providing a biological fluid sample from a subject having said disease and from a subject known not to have the disease (healthy)
- Directly quantifying circulating cell free DNA (ccfDNA)
- Extracting ccfDNA
- Treating the said DNA to convert demethylated cytosines to uracils while sparing the methylated cytosines
- Performing a methylation specific detection to measure diabetes-related gene methylation
- Incorporate the said methylation measurements of diabetes-related genes to the trained machine learning tool as the input parameters.

2. A method according to claim 1, wherein the said diabetes-related genes are pancreatic β-cell genes and preferably genes *GSK* and/or *IAPP* and/or *KCNJ11* and combinations thereof.

3. A method according to any of the previous claims, wherein said machine learning model has an AUC of 0.884 or higher.

4. A method according to any of the previous claims, wherein said machine learning tool is using one of the following algorithms: Decision Tree, k-Nearest Neighbors (k-NN), Gradient Boosting Machine (GBM), linear kernel Support Vector Machine (SVM-linear), Radial Basis Function (RBF) kernel Support Vector Machine, Artificial Neural Network (ANN), Multifactor Dimensionality Reduction (MDR), naive Bayes, Classification And Regression Tree (CART) and preferably Support Vector Machine (SVM) or (Classification) Random Forest (RF) or Logistic Regression (LR).

5. A method according to any of the previous claims, wherein said methylation measurements are expressed either qualitatively or quantitatively as indexes of methylation levels and preferably calculated by any of the following quantification methods/formulas and combinations thereof:

- The relative quantity (RQsample) of demethylated alleles using the $2^{-\Delta\Delta CT}$ method where the $\Delta\Delta CT$ values were generated for each target after normalization by reference gene values.
- The demethylation index was calculated using the formula: $2^{(\text{methylated Ct} - \textit{unmethylated Ct})}$.
- The percentage of methylation in a sample using the formula: $1 - \dfrac{1}{1+2^{(-\Delta Ct)}} \times 100\%$, where $\Delta Ct = Ct_{\text{unmethylated}} - Ct_{\text{methylated}}$

6. A method according to any of the previous claims, wherein the reference gene is any housekeeping gene and preferably *ACTB* or *GADPH* or *COL2A1* gene.

7. A method according to any of the previous claims, wherein the biological sample comprises a body fluid and preferably blood and more preferably serum or plasma.

8. A method according to any of the previous claims, wherein the methylation specific detection to measure diabetes-related gene methylation levels is conducted by sequencing or by PCR-based technology.

9. A method according to any of the previous claims, wherein the input parameters further comprising lifestyle and/or personal and/or demographic and/or clinical and/or clinicopathological data of the subjects and more preferably the BMI and/or the smoking status and /or the age of the subjects and combinations thereof.

10. A method for monitoring pancreatic β-cell destruction indicative of disease prediction, prognosis and/or diagnosis of T2DM, said method comprising

- Providing a biological fluid sample from a subject suspected of having or having said disease

- Directly quantifying circulating cell free DNA (ccfDNA)
- Extracting ccfDNA
- Treating the said DNA to convert demethylated cytosines to uracils while sparing the methylated cytosines
- Performing a methylation specific detection based on PCR or sequencing-based technology and preferably quantitative methylation specific PCR, to measure the methylation of diabetes-related genes and preferably pancreatic β-cell genes and more preferably of the genes *GSK* and/or *IAPP* and/or *KCNJ11* and combinations thereof
- Incorporate input data comprising of measurements of methylation of at least two of the said selected genes as data input parameters into the trained predictive model built by predictive machine learning tool of any of the previous claims 1 to 9
- Produce a score using the trained machine learning tool of any of the previous claims 1 to 9, indicative for clinical prediction, prognosis or diagnosis.

11. A method according to claim 10, wherein the lifestyle and/or personal and/or demographic data and/or clinical and/or clinicopathological data of the subject are further incorporated as input data in the tool and more preferably the BMI and/or the smoking status and /or the age of the subjects and combinations thereof.

12. A method according to any of the previous claims 10, 11, wherein said methylation measurements are expressed either qualitatively or quantitatively as indexes of methylation levels and preferably calculated by any of the following quantification methods/formulas and combinations thereof

- The relative quantity (RQsample) of demethylated alleles using the $2^{-\Delta\Delta CT}$ method where the $\Delta\Delta CT$ values were generated for each target after normalization by reference gene values.
- The demethylation index was calculated using the formula: $2^{(\text{methylated Ct}-\text{unmethylated Ct})}$.

- The percentage of methylation in a sample using the formula: $1 - \dfrac{1}{1+2^{(-\Delta Ct)}} \times 100\%$, where $\Delta Ct = Ct_{\text{unmethylated}} - Ct_{\text{methylated}}$

13. A method according to any of the previous claims 10-12, wherein the biological sample comprises body fluid and preferably blood sample and more preferably serum or plasma.

FIG 1

FIG 2

FIG 3

FIG 4

FIG 5

Europäisches
Patentamt

European
Patent Office

Office européen
des brevets

# EUROPEAN SEARCH REPORT

Application Number

EP 21 38 6030

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | HANG LAI ET AL: "Predictive models for diabetes mellitus using machine learning techniques", BMC ENDOCRINE DISORDERS, BIOMED CENTRAL LTD, LONDON, UK, vol. 19, no. 1, 15 October 2019 (2019-10-15), pages 1-9, XP021273082, DOI: 10.1186/S12902-019-0436-6 * the whole document * * in particular * * "methods" and "results" sections * | 1-13 | INV. G16H50/20 G16B30/00 G16B40/20 G16H50/70 |
| Y | FAROOQ SYED ET AL: "Circulating unmethylated and DNA fragments provide evidence of possible islet cell death in youth with obesity and diabetes", CLINICAL EPIGENETICS, BIOMED CENTRAL LTD, LONDON, UK, vol. 12, no. 1, 31 July 2020 (2020-07-31), pages 1-15, XP021279960, ISSN: 1868-7075, DOI: 10.1186/S13148-020-00906-5 * the whole document * * in particular * * "methods", "discussion" and "results" sections; figures 2, 6, 7 * | 1-13 | |

TECHNICAL FIELDS SEARCHED (IPC)

G16H
G16B

-/--

The present search report has been drawn up for all claims

| Place of search | Date of completion of the search | Examiner |
|---|---|---|
| Munich | 19 November 2021 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 21 38 6030

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | KAUR HARLEEN ET AL: "Predictive modelling and analytics for diabetes using a machine learning approach", APPLIED COMPUTING AND INFORMATICS, [Online] vol. ahead-of-print, no. ahead-of-print, 28 July 2020 (2020-07-28), XP055863324, ISSN: 2210-8327, DOI: 10.1016/j.aci.2018.12.004 Retrieved from the Internet: URL:https://www.emerald.com/insight/content/doi/10.1016/j.aci.2018.12.004/full/pdf?title=predictive-modelling-and-analytics-for-diabetes-using-a-machine-learning-approach> [retrieved on 2021-11-18] * the whole document * * in particular * * "material and method" section * ----- | 1-13 | |
| Y | SANABILALIHASSAN AHMED ET AL: "The role of DNA methylation in the pathogenesis of type 2 diabetes mellitus", CLINICAL EPIGENETICS, BIOMED CENTRAL LTD, LONDON, UK, vol. 12, no. 1, 11 July 2020 (2020-07-11), pages 1-23, XP021279183, ISSN: 1868-7075, DOI: 10.1186/S13148-020-00896-4 * the whole document * * in particular * * pages 8, 11, 13; "conclusion" section; table 1 * ----- -/-- | 1-13 | TECHNICAL FIELDS SEARCHED (IPC) |

The present search report has been drawn up for all claims

| Place of search | Date of completion of the search | Examiner |
|---|---|---|
| Munich | 19 November 2021 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 21 38 6030

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | Tsamardinos Ioannis ET AL: "Just Add Data: Automated Predictive Modeling and BioSignature Discovery", bioRxiv, 5 May 2020 (2020-05-05), pages 1-46, XP055863366, DOI: 10.1101/2020.05.04.075747 Retrieved from the Internet: URL:https://www.biorxiv.org/content/10.1101/2020.05.04.075747v1.full.pdf [retrieved on 2021-11-18] * the whole document * ----- | 1-13 | |

TECHNICAL FIELDS
SEARCHED (IPC)

The present search report has been drawn up for all claims

| Place of search | Date of completion of the search | Examiner |
|---|---|---|
| Munich | 19 November 2021 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160369340 A1
- US 20210087630 A1
- US 20170308981 A1, Blecker Saul

**Non-patent literature cited in the description**

- **SAEEDI P ; PETERSOHN I ; SALPEA P ; MALANDA B ; KARURANGA S ; UNWIN N et al.** Global and regional diabetes prevalence estimates for 2019 and projections for 2030 and 2045: Results from the International Diabetes Federation Diabetes Atlas. *Diabetes research and clinical practice,* 2019, 157
- **KHARROUBI AT ; DARWISH HM.** Diabetes mellitus: The epidemic of the century. *World J Diabetes,* 2015, vol. 6 (6), 850-67
- **RAHIER J ; GUIOT Y ; GOEBBELS RM ; SEMPOUX C ; HENQUIN JC.** Pancreatic beta-cell mass in European subjects with type 2 diabetes. *Diabetes, obesity & metabolism,* 2008, vol. 10 (4), 32-42
- **BUTLER AE ; JANSON J ; BONNER-WEIR S ; RITZEL R ; RIZZA RA ; BUTLER PC.** Beta-cell deficit and increased beta-cell apoptosis in humans with type 2 diabetes. *Diabetes,* 2003, vol. 52 (1), 102-10
- **AKIRAV EM ; LEBASTCHI J ; GALVAN EM ; HENEGARIU O ; AKIRAV M ; ABLAMUNITS V et al.** Detection of beta cell death in diabetes using differentially methylated circulating DNA. *Proceedings of the National Academy of Sciences of the United States of America,* 2011, vol. 108 (47), 19018-23
- **HUSSEINY MI ; KAYE A ; ZEBADUA E ; KANDEEL F ; FERRERI K.** Tissue-specific methylation of human insulin gene and PCR assay for monitoring beta cell death. *PloS one,* 2014, vol. 9 (4), e94591
- **HEROLD KC ; USMANI-BROWN S ; GHAZI T ; LEBASTCHI J ; BEAM CA ; BELLIN MD et al.** beta cell death and dysfunction during type 1 diabetes development in at-risk individuals. *The Journal of clinical investigation,* 2015, vol. 125 (3), 1163-73
- **OLSEN JA ; KENNA LA ; SPELIOS MG ; HESSNER MJ ; AKIRAV EM.** Circulating Differentially Methylated Amylin DNA as a Biomarker of beta-Cell Loss in Type 1 Diabetes. *PloS one,* 2016, vol. 11 (4), e0152662

- **SKLENAROVA J ; PETRUZELKOVA L ; KOLOUSKOVA S ; LEBL J ; SUMNIK Z ; CINEK O.** Glucokinase Gene May Be a More Suitable Target Than the Insulin Gene for Detection of beta Cell Death. *Endocrinology,* 2017, vol. 158 (7), 2058-65
- **LEHMANN-WERMAN R ; NEIMAN D ; ZEMMOUR H ; MOSS J ; MAGENHEIM J ; VAKNIN-DEMBINSKY A et al.** Identification of tissue-specific cell death using methylation patterns of circulating DNA. *Proceedings of the National Academy of Sciences of the United States of America,* 2016, vol. 113 (13), E1826-34
- **USMANI-BROWN S ; LEBASTCHI J ; STECK AK ; BEAM C ; HEROLD KC ; LEDIZET M.** Analysis of beta-cell death in type 1 diabetes by droplet digital PCR. *Endocrinology,* 2014, vol. 155 (9), 3694-8
- **FISHER MM ; PEREZ CHUMBIAUCA CN ; MATHER KJ ; MIRMIRA RG ; TERSEY SA.** Detection of islet beta-cell death in vivo by multiplex PCR analysis of differentially methylated DNA. *Endocrinology,* 2013, vol. 154 (9), 3476-81
- **GAHAN PB.** Biology of circulating nucleic acids and possible roles in diagnosis and treatment in diabetes and cancer. *Infectious disorders drug targets,* 2012, vol. 12 (5), 360-70
- **BERGMAN Y ; CEDAR H.** DNA methylation dynamics in health and disease. *Nature structural & molecular biology,* 2013, vol. 20 (3), 274-81
- **DAVENPORT T ; KALAKOTA R.** The potential for artificial intelligence in healthcare. *Future healthcare journal,* 2019, vol. 6 (2), 94-8
- **MYSZCZYNSKA MA ; OJAMIES PN ; LACOSTE AMB ; NEIL D ; SAFFARI A ; MEAD R et al.** Applications of machine learning to diagnosis and treatment of neurodegenerative diseases. *Nature Reviews Neurology,* 2020, vol. 16 (8), 440-56
- **SCHAEFER J ; LEHNE M ; SCHEPERS J ; PRASSER F.** Thun S. The use of machine learning in rare diseases: a scoping review. *Orphanet Journal of Rare Diseases,* 2020, vol. 15 (1), 145

- **KOUROU K ; EXARCHOS TP ; EXARCHOS KP ; KARAMOUZIS MV ; FOTIADIS DI.** Machine learning applications in cancer prognosis and prediction. *Computational and Structural Biotechnology Journal,* 2015, vol. 13, 8-17
- **ZHANG L ; WANG Y ; NIU M ; WANG C ; WANG Z.** Machine learning for characterizing risk of type 2 diabetes mellitus in a rural Chinese population: the Henan Rural Cohort Study. *Scientific Reports,* 2020, vol. 10 (1), 4406
- **MUHAMMAD LJ ; ALGEHYNE EA ; USMAN SS.** Predictive Supervised Machine Learning Models for Diabetes Mellitus. *SN Computer Science,* 2020, vol. 1 (5), 240
- **KARAGLANI M ; GOURLIA K ; TSAMARDINOS I ; CHATZAKI E.** Accurate Blood-Based Diagnostic Biosignatures for Alzheimer's Disease via Automated Machine Learning. *Journal of clinical medicine,* September 2020, vol. 9 (9
- **MARKAKI M ; TSAMARDINOS I ; LANGHAMMER A ; LAGANI V ; HVEEM K ; RØE OD.** A Validated Clinical Risk Prediction Model for Lung Cancer in Smokers of All Ages and Exposure Types: A HUNT Study. *EBioMedicine,* 2018, vol. 31, 36-46
- **PANAGOPOULOU M ; KARAGLANI M ; BALGKOURANIDOU I ; BIZIOTA E ; KOUKAKI T ; KARAMITROUSIS E et al.** Circulating cell-free DNA in breast cancer: size profiling, levels, and methylation patterns lead to prognostic and predictive classifiers. *Oncogene,* 2019, vol. 38 (18), 3387-401
- **AMERICAN DIABETES ASSOCIATION.** Classification and Diagnosis of Diabetes. *Diabetes Care,* 2015, vol. 38 (1), S8
- **HATTERMANN K ; MEHDORN HM ; MENTLEIN R ; SCHULTKA S ; HELD-FEINDT J.** A methylation-specific and SYBR-green-based quantitative polymerase chain reaction technique for O6-methylguanine DNA methyltransferase promoter methylation analysis. *Analytical biochemistry,* 2008, vol. 377 (1), 62-71
- **LI LC ; DAHIYA R.** MethPrimer: designing primers for methylation PCRs. *Bioinformatics,* 2002, vol. 18 (11), 1427-31
- **LU L ; KATSAROS D ; DE LA LONGRAIS IA ; SOCHIRCA O ; YU H.** Hypermethylation of let-7a-3 in epithelial ovarian cancer is associated with low insulin-like growth factor-II expression and favorable prognosis. *Cancer research,* 2007, vol. 67 (21), 10117-22
- **LIVAK KJ ; SCHMITTGEN TD.** Analysis of relative gene expression data using real-time quantitative PCR and the 2(-Delta Delta C(T)) Method. *Methods,* 2001, vol. 25 (4), 402-8
- **TSAMARDINOS I ; CHARONYKTAKIS P ; LAKIOTAKI K ; BORBOUDAKIS G ; ZENKLUSEN JC ; JUHL H et al.** Just Add Data: Automated Predictive Modeling and BioSignature Discovery. *bioRxiv*
- **FARRAN B ; CHANNANATH AM ; BEHBEHANI K ; THANARAJ TA.** *Predictive models to assess risk of type 2 diabetes, hypertension and comorbidity: machine-learning algorithms and validation using national health data from Kuwait—a cohort study,* 2013, vol. 3 (5), e002457
- **LAI H ; HUANG H ; KESHAVJEE K ; GUERGACHI A ; GAO X.** Predictive models for diabetes mellitus using machine learning techniques. *BMC Endocrine Disorders,* 2019, vol. 19 (1), 101
- **KAUR H ; KUMARI V.** Predictive modelling and analytics for diabetes using a machine learning approach. *Applied Computing and Informatics,* 2020